# EUROPEAN PATENT SPECIFICATION

(11) **EP 1 336 389 B1**
(45) Date of publication and mention of the grant of the patent: **26.04.2006**
(21) Application number: 03003131.4
(22) Date of filing: 13.02.2003
(51) Int. Cl.: A61C 13/15

(54) **Light irradiation apparatus for dental photo polymerization composite resin**
Lichtbestrahlungsgerät zur Fotopolymerisation von dentalen Verbundharzen
Appareil de photopolymérisation de résines composites dentaires

(30) Priority: 14.02.2002 JP 2002037056
(43) Date of publication of application: 20.08.2003
(73) Proprietor: GC Corporation, Itabashi-ku, Tokyo 174 (JP)
(72) Inventor: Kumagai, Tomohiro, GC Corporation, Tokyo (JP); Fusejima, Futoshi, GC Corporation, Tokyo (JP); Takada, Mitsuaki, GC Corporation, Tokyo (JP); Kobayashi, Aiichi, GC Corporation, Tokyo (JP); Yamaguchi, Kaoru, GC Corporation, Tokyo (JP)
(74) Representative: Müller-Boré & Partner Patentanwälte

(56) References cited:
- WO-A-00/67048
- US-A- 6 099 520
- US-B1- 6 282 013

## Description

The present invention relates to a light irradiation apparatus for a dental photo polymerization composite resin which can well polymerize and cure the dental photo polymerization composite resin corresponding to a subject to be irradiated by irradiating an irradiation light having a specific wavelength which is selected in correspondence to a characteristic of a photo polymerization catalyst of the dental photo polymerization composite resin employed for a dental remedy.

The dental photo polymerization composite resin is going to achieve a great demand in clinicians, because it is excellent in points that an aspect which has no uncomfortable feeling against natural teeth can be easily applied (operability) and that a color tone close to the natural teeth can be obtained (aesthetic characteristic), in addition to an improvement in performance in recent years. Such an enhancement in demand not only brings a high performance of the dental photo polymerization composite resin itself, but also prompts a development in peripheral devices for using the material.

Previously, as the light irradiation apparatus for polymerizing and curing the dental photo polymerization composite resin which is filled in a tooth within an oral cavity of a patient, there is employed an apparatus in which an outer shape is formed as if a hair dryer, a halogen lamp or a xenon lamp corresponding to a light source is received in an inner portion thereof, and a columnar light guide (which generally employs a bundle of glass fibers) which is formed by a transparent material for introducing a light irradiating to the dental photo polymerization composite resin filled in the subject tooth can be attached to a leading end of the apparatus.

In the conventional light irradiation apparatus, there is no alternative but to employ the structure mentioned above, because of the following reasons.

First, as a light source which can obtain a necessary and sufficient light for polymerizing and curing the dental photo polymerization composite resin, the halogen lamp and the xenon lamp are the most suitable. However, since these light sources also generate a large amount of heat together at a time of lighting, it is necessary to cool in order to avoid deterioration not only of a service life of the lamp itself but also of the apparatus itself.

Accordingly, since a cooling mechanism for the light source in accordance with a ventilation using a cooling fan is essential, the light irradiation apparatus can not be made smaller than a certain size. Further, since an electric power consumption is great as a matter of course, there exists a constraint condition that it is unavoidable to receive an electricity supply by using an electric wire. In addition, a great transformer is required for providing for the great electric power consumption of the light source, and the like. As mentioned above, it is necessary to take into consideration storage and arrangement of the peripheral functional parts for the purpose of providing a target function.

Accordingly, in the light irradiation apparatus which uses the halogen lamp or the xenon lamp as the light source, it is impossible to downsize the apparatus for the purpose of improving usability thereof, it is impossible to make the cooling fan generating a noise unnecessary so as to give no pain caused by the noise to the patient, it is impossible to make the structure cordless so as to eliminate the negative effect to the medical treatment by the electric wire supplying the electricity to the light source, and it is impossible to freely select a length of the light guide (since there is a risk that the cooling wind becoming a hot wind is directly blown on the face of the patient if the length of the light guide is short, it is necessary that a certain fixed length (distance) is secured). Accordingly, there is a condition that it is impossible in view of the structure to form the light irradiation apparatus which is light in weight and excellent in an operability.

In this case, in order to photo polymerize the dental photo polymerization composite resin which is frequently employed at present, a combination of a camphor quinone and a tertiary amine is most frequently used as the photo polymerization catalyst. Then, the characteristic of the camphor quinone becomes apparent as a result of research effort of many researchers. That is, it is known that an optical wavelength band in which the camphor quinone sympathizes most efficiently absorbs a light having 470 nm as a peak and being in the range between 20 and 30 nm of the peak. This wavelength band is a visible light, and the light in a blue band.

On the other hand, since the halogen lamp, for example, used as the light source irradiates the light in the wide light wavelength band between 350 and 800 nm, the light having the wavelength necessary for photo polymerizing the dental photo polymerization composite resin is taken out from the light by interposing a wavelength selection filter so as to use the light. At this time, it is said that a luminous intensity (brightness) is attenuated to about one tenth due to using of this wavelength selection filter. Further, not only this halogen lamp is significantly reduced in a quantity of light (the quantity of light becomes 60 to 70 % for about 25 hours) even by a normal use, but also a service life of the halogen lamp is not so long (about 50 hours). Further, since a transient current forming a great load flows at a time of being turned on, it has a weakness for flashing. That is, there is a disadvantage that its own service life is shortened in each case that the lamp flashes. Accordingly, in order to make the dental photo polymerization composite resin perform a complete photo polymerization, it is necessary to always pay attention to the lamp service life and the use state.

However, since an LED generating a blue light was developed about 1994 and 1995, there are performed various ingenuity and proposals for assembling the LED in the light irradiation apparatus so as to use. As a representative example, there is a "photo polymerization resin curing light source apparatus" constituted of a plurality of light emitting diodes having a peak emission wavelength in the range of 430 to 480 nm, an optical means to condense the light from the diodes, and a light irradiation means to output the light condensed by the optical means (see Patent Document 1 below for an example), which has been also reported as a research document in the association concerned (see Non-patent Document 1 below).

On the basis of such research document etc. and in accordance with an increase in the performance of the LED, the light irradiation apparatus actually employing a high intensity blue LED is sold by foreign and domestic companies as a product, and is going to be used in an actual clinical case. In catalogues of these products, there is emphasized that the technical problem which can not be solved because the light source mentioned above is the lamp can be cleaned up. That is, there are described advantages that the apparatus is compact and light and is cordless, an electric power consumption is small, the apparatus is silent, the service life of the light source is semi-permanent and the like. As mentioned above, the light irradiation apparatus for photo polymerizing the dental photo polymerization composite resin makes rapid progress and evolution.

### Patent Document 1:

The publication of Japanese Patent No. 2976522.

### Non-patent Document 1:

"Research relating,to irradiator making use of blue light emitting diodes" by Fujibayashi, et al. reported in Japan Conservative Dentistry Association Journal, No. 1, volume 39.

on the contrary, the dental photo polymerization composite resin which is to be photo polymerized is not changed at all. That is, the combination of the camphor quinone and the tertiary amine is continuously employed as the catalyst for the photo polymerization. However, in the camphor quinone, there can be pointed out the fact that the color tone of the camphor quinone is yellow (this yellow is preferable because the healthy natural tooth is similar to yellow), however, the yellow color fades after being polymerized and cured in accordance with the light irradiation, and the remaining yellow color further fades in accordance with an elapse of time, as a peculiar nature. Therefore, there is a disadvantage that the color becomes different from the natural tooth at a polymerizing and curing stage, even when a color matching is performed in view of the aesthete dentistry hardly.

Further, since the combination with the tertiary amine is used, there is a defect that a keeping quality of the material is rather difficult (there is a risk that the unused dental photo polymerization composite resin changes in quality due to an elapse of time). In order to cope with such a defect, there has been expected that the dental photo polymerization composite resin using a new photo polymerization initiator auxiliary agent is researched and developed.

In this case, another complaint is generated by the clinical dentist who uses the light irradiation apparatus on which the high intensity blue LED is mounted as the light source mentioned above. It is the matter that since the photo polymerization by the high intensity blue LED has a shallow case depth (a depth from a surface in which the polymerization and curing is sufficiently performed) of the dental photo polymerization composite resin and a much non-polymerized portion in comparison with the photo polymerization by the previous halogen lamp, it lacks a reliability as a prosthetic appliance to the tooth in view of maintaining. That is, there frequently occurs a phenomenon that the composite resin drops out from the tooth for a short period after the medical treatment.

Further, in order to cope with the color fading of the camphor quinone mentioned above (the inherent yellow color fades out), there has been in recent years developed a dental photo polymerization composite resin using an acylphosphine oxide [a trade name: Luciline TPO (manufactured by BASF company) and the like] as a photo polymerization initiator which does not require the photo polymerization initiator auxiliary agent. Since the photo polymerization initiator is transparent and colorless, the aesthetic color matching can be easily performed (a color measurement can be performed only by the inherent color of the resin and no color fading is generated), so as to finish according to an imaged color. Further, the photo polymerization initiator is a material in which a sympathizing optical wavelength range is peaked at 380 nm, as is different from the camphor quinone in which a sympathizing optical wavelength range is peaked at 470 nm.

However, since all of the light irradiation apparatuses for the dental photo polymerization composite resin which use the currently available LED as the light source are designed for the purpose of being applicable only to the camphor quinone, the apparatus uses only a blue ray radiation LED having a peak of luminous wavelength in 450 to 490 nm. Accordingly, it is impossible to solve the defect mentioned above that the case depth of the dental photo polymerization composite resin is shallow and the non-polymerized portion is much, and the defect that it is impossible to be used for the dental photo polymerization composite resin which uses the acylphosphine oxide as the photo polymerization initiator developed newly in recent years.

Document US 6,282,013 B1 describes a method and system for curing polymeric materials including dental composites. Preferably, modulated light is used to control the formation of polymer chains in the polymeric materials so that a cured polymeric material that has the desired physical characteristics for its intented function. Formation of short chain and long chain polymers from monomers in the polymeric material is initiated and controlled by using a light source with a wave length suitable for one or more initiators found in the polymeric material.

Document WO 00/67048 describes an optical device including a diode light source which is preferably in the form of an array of diode elements such as laser diodes or light emitting diodes. The diode element preferably emit in either the blue or ultraviolet region of the optical spectrum.

An object of the present invention is to provide a light irradiation apparatus for a dental photo polymerization composite resin of multi-purpose type which can solve the defect in the conventional light irradiation apparatus for the dental photo polymerization composite resin, can efficiently and completely polymerize and cure the dental photo polymerization composite resin using a combination of the camphor quinone and the tertiary amine as the photo polymerization catalyst in accordance with a light irradiation, and can also polymerize and cure the dental photo polymerization composite resin using the acylphosphine oxide as the newly developed photo polymerization initiator in accordance with the light irradiation.

The inventors of the present application devote themselves to research so as to solve the problems mentioned above. As a result, the inventors presumes that there is generated the phenomenon that the non-polymerized portion is left in the dental photo polymerization composite resin using the combination of the camphor quinone and the tertiary amine as the photo polymerization catalyst because there is only used the high intensity blue LED corresponding only to the optical wavelength range to be sympathized by the camphor quinone having a peak at 470 nm and a spread of 20 to 30 nm before and af ter thereof, and then executes an experiment for confirming how the case depth is, by combining various LEDs which generate the lights in the other wavelength ranges. As a result, the inventors find that a very preferable result can be obtained by combining with near ultraviolet rays and/or violet rays radiation LED having luminous wavelengths peaked at a wavelength of 370 nm or more but less than 410 nm, and the light irradiated from the near ultraviolet rays and/or violet rays radiation LED can be made use of polymerizing and curing the dental photo polymerization composite resinusing the acylphosphine oxide as the newly developed photo polymerization initiator on the basis of the light irradiation. Accordingly, the inventors complete the present invention.

That is, in accordance with the present invention, there is provided a light irradiation apparatus for a dental photo polymerization composite resin comprising:
a blue ray radiation LED having luminous wavelengths peaked at a wavelength of 450 nm or more but not more than 490 nm;
a near ultraviolet rays and/or violet rays radiation LED having luminous wavelengths peaked at a wavelength of 370 nm or more but less than 410 nm; and
a circuit changing switch at least for simultaneously turning on these two kinds of LEDs and for turning on only the near ultraviolet rays and/or violet rays radiation LED.

Further, in the light irradiation apparatus for the dental photo polymerization resin mentioned above, the structure may be made such that there are provided a switch for selecting a lighting time of the light rays radiation LEDs from a plurality of preset times and/or a time setting switch capable of setting the lighting time to an optional time. In this case, since the light can be irradiated in a state of previously matching to a specified time for light irradiation of each of the dental photo polymerization composite resins which are respectively sold by a plurality of makers, a labor hour can be reduced and the irradiating time can be set in correspondence to an amount of use of the dental photo polymerization composite resin, whereby a preferable result can be obtained. The inventors also find the above fact.

Further, in the light irradiation apparatus for the dental photo polymerization resin mentioned above, the structure may be made such that a filter for shutting off the ultraviolet rays having a specific wavelength or less is arranged between the light rays radiation LEDs and a light irradiation port. In this case, it is possible to effectively and securely remove the improper ultraviolet rays having short wavelengths which have a cancer-causing effect and are deemed to be harmful to the human body, whereby a preferable result can be obtained. The inventors also find the above fact.

Further, in the light irradiation apparatus for the dental photo polymerization resin mentioned above, the structure may be made such that a ratio in output between the blue rays radiation LED and the near ultraviolet rays and/or violet rays radiation LED is 9 : 1 to 5 : 5. In this case, it is possible to solve the disadvantage that the non-polymerized portion is left at a time of polymerizing and curing not only the dental photo polymerization composite resin using the acylphosphine oxide as the photo polymerization initiator, but also the dental photo polymerization composite resin using the combination of the camphor quinone and the tertiary amine in accordance with the light irradiation so as to obtain a complete polymerized and cured product, and a light irradiation time can be made short, whereby a preferable result can be obtained.

Fig. 1 is a cross sectional schematic view which describes a structure of an embodiment of a light irradiation apparatus for a dental photo polymerization composite resin in accordance with the present invention;

Fig. 2 is a perspective schematic view showing a state in which the light irradiation apparatus for the dental photo polymerization composite resin in accordance with the present invention shown in Fig. 1 is mounted on a charging stand;

Fig. 3 is a block circuit diagram which described a general structure of an electric circuit in the light irradiation apparatus for the dental photo polymerization composite resin in accordance with the present invention; and

Fig. 4 is a spectral characteristic schematic view which describes an emission spectral characteristic of a light rays radiation LED used in the light irradiation apparatus for the dental photo polymerization composite resin in accordance with the present invention.

A description will be given below of details of an embodiment of a light irradiation apparatus for a dental photo polymerization composite resin in accordance with the present invention with reference to the accompanying drawings. However, the present invention is not limited to the following embodiment.

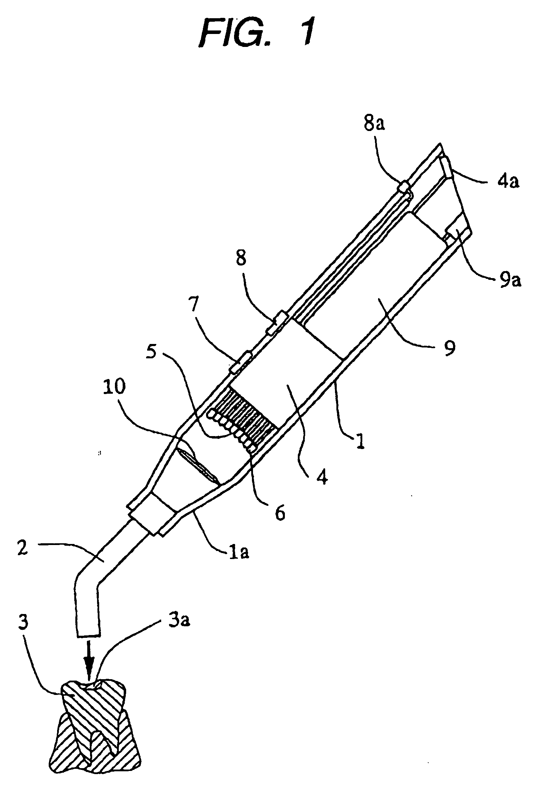

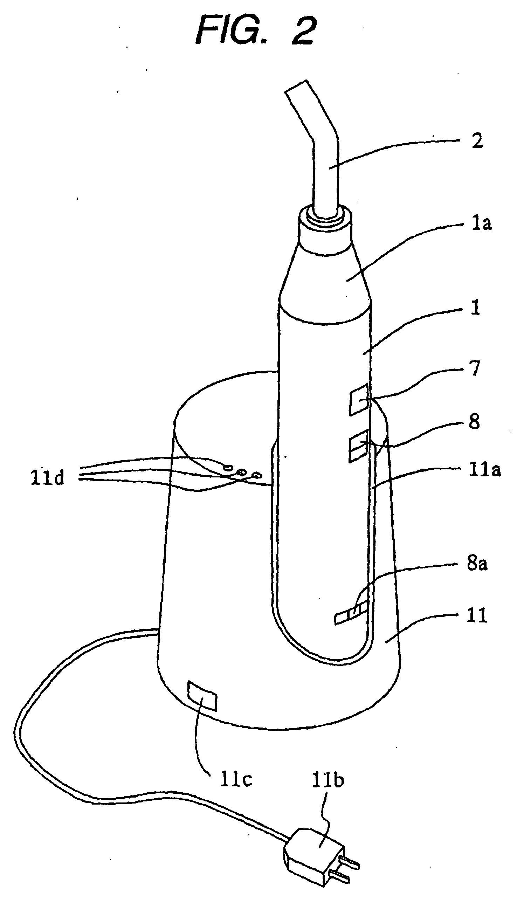

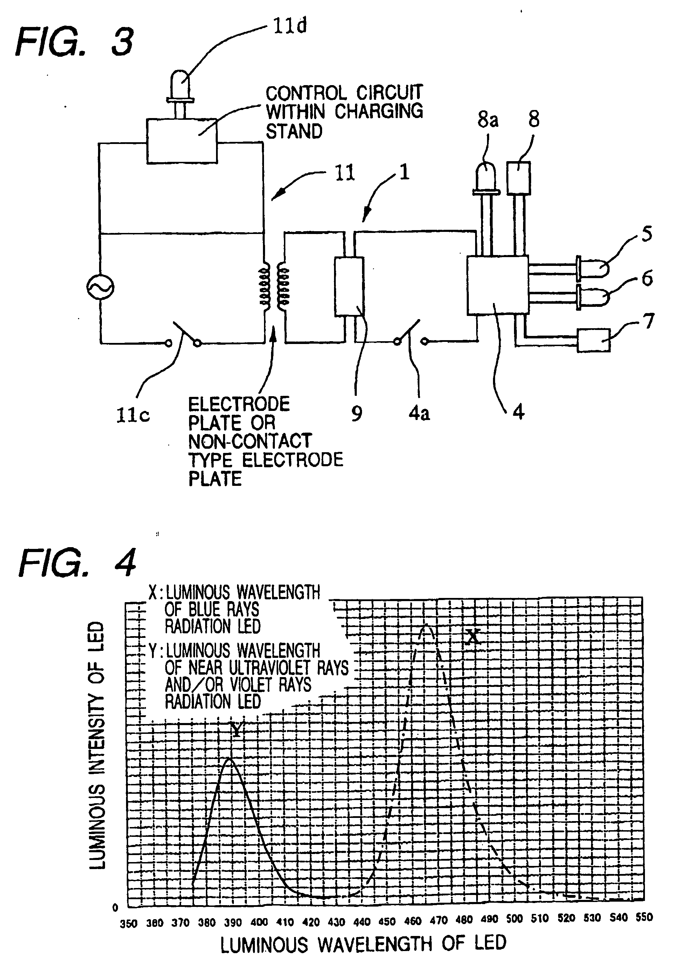

Fig. 1 is a cross sectional schematic view which describes a structure of an embodiment of a light irradiation apparatus for a dental photo polymerization composite resin in accordance with the present invention, Fig. 2 is a perspective schematic view showing a state in which the light irradiation apparatus for the dental photo polymerization composite resin in accordance with the present invention shown in Fig. 1 is mounted on a charging stand, Fig. 3 is a block circuit diagram which described a general structure of an electric circuit in the light irradiation apparatus for the dental photo polymerization composite resin in accordance with the present invention, and Fig. 4 is a spectral characteristic schematic view which describes an emission spectral characteristic of a light rays radiation LED used in the light irradiation apparatus for the dental photo polymerization composite resin in accordance with the present invention.

In the drawings, reference numeral 1 denotes a light irradiator main body formed in a substantially columnar shape in which a leading end portion 1a is formed in a tapered shape and a maximum diameter is about 35 to 45 mm so as to be easily gripped by a person. The light irradiator main body 1 is wholly formed by a resin for the purpose of saving weight so as not to apply load due to the weight to the user. Otherwise, the light irradiator main body 1 may be formed by a light alloy at convenience of balance caused by a position of center of gravity or a design.

Reference numeral 2 denotes a light guide in which a rear end portion thereof is set in an insertion hole corresponding to a light irradiation port provided substantially in the center of the leading end portion 1a of the light irradiator main body 1, and optical transmission fibers such as glass optical fibers or plastic optical fibers or the like hardly causing optical transmission loss are bundled so as to be integrated. It is preferable that the leading end side of the light guide 2 is slightly curved as illustrated so as to easily irradiate the light to a dental photo polymerization composite resin 3a which is filled in a tooth 3 within a oral cavity as a prosthetic appliance. Further, in the case that a plurality of light guides 2 having different lengths are prepared in correspondence to portions in the tooth 3 which is the subject to be irradiated within the mouth cavity, a usability for the user is improved. Accordingly, this structure is preferable.

Reference numeral 4 denotes a control circuit which is put within the light irradiator main body 1. This control circuit 4 is on-off controlled by a main switch 4a which is arranged at the rear end of the light irradiator main body 1 basically as shown in Fig. 1. Further, the structure is made such that the control circuit 4 controls lighting times of light rays radiation LEDs 5 and 6 mentioned below or displays the set lighting times in response to an operation and setting of each of switches mentioned below, and in addition, it is a matter of course that a circuit indicating a remaining amount of an electric power of a battery 9 mentioned below which is previously installed within the light irradiator main body 1 may be built in the control circuit 4.

Reference numeral 5 denotes a blue rays radiation LED which is put within the light irradiator main body 1. Five to nine blue rays radiation LEDs are arranged in a lump near the axial center of the light irradiator main body 1. Luminous wavelengths of the blue rays radiation LED are peaked at a wavelength of 450 nm or more but not more than 490 nm. It is preferable to use a high intensity light rays radiation LED which is widely used in the present market.

Reference numeral 6 denotes a near ultraviolet rays and/or violet rays radiation LED. Four to ten near ultraviolet rays and/or violet rays radiation LEDs are arranged around the periphery of the blue rays radiation LEDs 5 mentioned above. Luminous wavelengths of the near ultraviolet rays and/or violet rays radiation LED are peaked at a wavelength of 370 nm or more but less than 410 nm. It is preferable to use a near ultraviolet rays and/or violet rays radiation LED which is widely used for irradiating to an anti-bacterial and deodorant member carrying a photo catalyst arranged in a refrigerator, an air conditioner and the like.

In a normal method in a dental remedy, the apparatus is used 1 mm to 2 mm apart from a surface of the tooth 3 which is the subject to be irradiated (the filled dental photo polymerization composite resin 3a). Further, in the case that the light is irradiated to a dental photo polymerization composite resin using an acylphosphine oxide and a dental photo polymerization composite resin using a combination of a camphor quinone and a tertiary amine as a photo polymerization initiator corresponding to a use subject of the light irradiation apparatus in accordance with the present invention, a polymerization and curing reaction rapidly occurs in comparison with the case that the light is irradiated to the dental photo polymerization composite resin using only the combination of the camphor quinone and the tertiary amine as the conventional photo polymerization catalyst. Accordingly, it is possible to achieve the object even when the output of the near ultraviolet rays and/or violet rays radiation is small. Therefore, the rate in total amount of the output of the light radiated from the blue rays radiation LED 5 and the output of the light radiated from the near ultraviolet rays and/or violet rays radiation LED 6 is sufficiently satisfied by about 9:1 to 5:5.

That is, in the case of polymerizing and curing the dental photo polymerization composite resin using two kinds of photo polymerization catalysts as mentioned above by using the light irradiation apparatus in accordance with the present invention, if a rate in total amount of the light outputs between the blue rays radiation LED 5 and the near ultraviolet rays and/or violet rays radiation LED 6 is set to about 9 : 1 to 5 : 5, it is possible to solve the disadvantage that the non-polymerized portion is left in the dental photo polymerization composite resin which is generated in the case of using the conventional LED light irradiation apparatus, whereby a complete polymerized and cured product can be obtained, and the light irradiation time can be made short. Accordingly, this structure is preferable.

Further, although an illustration is omitted, in some kinds of the dental photo polymerization composite resin 3a which is the subject to be irradiated of the light irradiation apparatus in accordance with the present invention, there is a product in which the polymerization and curing is more promoted in a state in which the product is warmed up by receiving the light rays in the side of long wavelength (green or red) (it is known that the light in the long wavelength side has an effect of warming up the object). Accordingly, some LEDs generating the green and/or red light rays may be arranged in an area in which the blue rays radiation LEDs 5 are arranged. In this case, an intended object can be achieved by using the LEDs generating the green and/or red light rays at a level of about 10 % of the total output. It is possible to get the goodness of the luminous wavelength characteristic in the halogen lamp which has a high reliability for polymerizing and curing the dental photo polymerization composite resin 3a, by making the structure of the LEDs arranged within the light irradiator main body 1 in the manner mentioned above.

Reference numeral 7 denotes a circuit changing switch which is arranged on an outer peripheral side surface positioned in a slightly front side from the middle of a total length of the light irradiator main body 1. The circuit changing switch 7 is at least used for simultaneously turning on the blue rays radiation LEDs 5 and the near violet rays and/or ultraviolet rays radiation LEDs 6 and turning on only the near ultraviolet rays and/or violet rays radiation LEDs 6. It is possible to irradiate the light in correspondence to the characteristic of the dental photo polymerization composite resin 3a which is used as the prosthetic appliance to be filled in the tooth 3, by operating the circuit changing switch 7. Further, it is preferable to provide a display portion for discriminating what wavelength of the light is to be irradiated by the operation of the circuit changing switch 7 at a predetermined position of the light irradiator main body 1.

Reference numeral 8 denotes a time setting switch which is arranged in the light irradiator main body 1 adjacent to the circuit changing switch 7. The time setting switch 8 is used for optionally selecting a time from preset times (for example, four kinds comprising 5 seconds, ten seconds, twenty seconds and forty seconds) for controlling the lighting time of the light rays radiation LEDs 5 and 6. In the case of this embodiment, it may be possible to set another new time than the standard times by suitably combining four kinds of times mentioned above in response to the pressing number of the time setting switch 8 (for example, set the lighting time of fifteen seconds by combining five seconds and ten seconds), and the structure is made such that the selected time or the set time is displayed by lighting or flashing of four time displaying LEDs 8a which are arranged in the outer periphery of the rear end of the light irradiator main body 1.

Reference numeral 9 denotes a battery which is put in a rear side within the light irradiator main body 1. The control circuit 4 is operated and an electric power for lighting the light rays radiation LEDs 5 and 6 is supplied by an electric power stored within the battery 9. In the illustrated embodiment, connecting electrodes 9a and 9a which are arranged so as to be positioned in the opposite side to the time displaying LEDs 8a at the rear end of the light irradiator main body 1 are respectively connected to electrodes of a charging stand 11 mentioned below, whereby the electric power is supplied to the battery 9. The electric power supply to the battery 9 may be structured such as to have no relevance to the main switch 4a mentioned above. Further, it goes without saying that the electric power may be stored by utilizing an induced electromotive force without using the connecting electrodes 9a and 9a which are exposed to an external portion.

Reference numeral 10 denotes a disc-like filter which is arranged so as to be positioned between an attachment portion of the light guide 2 forming a light irradiation port within the light irradiator main body 1 and the light rays radiation LEDs 5 and 6. The filter 10 is used for shutting off ultraviolet rays having a specific wavelength or less, particularly a short wavelength less than 370 nm for example which is deemed to be harmful to the human body due to a cancer-causing effect. It is preferable that the filter 10 is formed in a prism shape as shown in Fig. 1 because the nature of the light can be well utilized. This prism shape is constituted in detail by making a center portion opposing to the blue rays radiation LEDs 5 uniform in thickness and making a portion opposing to the near ultraviolet rays and/or violet rays radiation LEDs 6 tapered to become thin toward the end.

At a time of not using the light irradiator main body 1 and at a time of charging, the light irradiator main body 1 is set into an insertion groove 11a of the charging stand 11 so as to be stored, as shown in Fig. 2. At this time, the battery 9 is automatically charged by inserting a plug 11b into a.bayonet plug (not shown) of an AC power supply and turning on a charging stand main switch 11c. Further, it is preferable to arrange a charging state display LED 11d at an easily observable position of the charging stand 11 so that the charging state can be recognized at a glance, thereby understanding what percent the charging runs into in comparison with a full charge state.

Relations among the respective elements described above are described in summary in a block circuit diagram in Fig. 3 describing an outline structure of an electric circuit.

As described in detail above, the light irradiation apparatus for the dental photo polymerization composite resin in accordance with the present invention is provided with all the following effects. Since the halogen lamp and the xenon lamp for the light source are not used, the cooling fan against the heat generation is not required, whereby a quietness is achieved. A handling is convenient due to the cordless structure. It is not required to take into consideration a reduction of service life of the light source. In addition to the above, which are the characteristic of the existing LED light irradiation apparatus the light irradiation apparatus for the dental photo polymerization composite resin in accordance with the present invention has a nature capable of also securely polymerizing and curing the dental photo polymerization composite resin using the combination of the camphor quinone and the tertiary amine for the conventional photo polymerization catalyst. Further, the present invention provides an epoch-making light irradiation apparatus for the dental photo polymerization composite resin which can respond to the polymerization and curing of the dental photo polymerization composite resin using the acylphosphine oxide for the photo polymerization initiator which is newly developed in recent years, and contributes to an improvement of technique in the dental remedy.

## Claims

1. A light irradiation apparatus polymerizing and caring mainly a dental photo polymerization composite resin using a combination of a camphor quinone and a tertiary amine or an acylphosphine oxide as the photo polymerization catatyst **characterized in that** it comprises:
a blue ray radiation LED 5 having luminous wavelengths peaked at a wavelength of 450 nm or more but not more than 490 nm;
a near ultraviolet rays and/or violet rays radiation LED 6 having luminous wavelengths peaked at a wavelength of 370 nm or more but less than 410 nm; and
a circuit changing switch 7 for simultaneously turning on these two kinds of LEDs 5, 6 and for turning on only the near ultraviolet rays and/or violet rays radiation LED 6.

2. The light irradiation apparatus for a dental photo polymerization resin as claimed in claim 1, wherein there are provided a switch 8 for selecting a lighting time of the light rays radiation LEDs 5, 6 from a plurality of preset times and/or a time setting switch capable of setting the lighting time to an optional time.

3. The light irradiation apparatus for a dental photo polymerization resin as claimed in claim 1 or 2, wherein a filter 10 for shutting off the ultraviolet rays having a specific wavelength or less is arranged between the light rays radiation LEDs 5, 6 and a light irradiation port.

4. The light irradiation apparatus for a dental photo polymerization resin as claimed in any one of claims 1 to 3, wherein a ratio in output between the blue rays radiation LED 5 and the near ultraviolet rays and/or violet rays radiation LED 6 is 9 : 1 to 5 : 5.

## Revendications

1. Dispositif de rayonnement de lumière assurant principalement la polymérisation et le durcissement d'une résine composite dentaire à photopolymérisation, utilisant un mélange de camphre quinone et d'une amine tertiaire ou d'un oxyde d'acylphosphine pour le catalyseur de photopolymérisation, **caractérisé en ce qu'**il comprend:
une diode LED rayonnant dans le bleu (5) présentant des longueurs d'onde lumineuses de crête à une longue d'onde supérieure ou égale à 450 nm mais non supérieure à 490 nm ;
une diode LED rayonnant dans le proche ultraviolet et/ou dans le violet (6) présentant des longueurs d'onde lumineuses de crête à une longueur d'onde supérieure ou égale à 370 nm mais inférieure à 410 nm ; et
un commutateur de changement de circuit (7) destiné à activer simultanément ces deux types de diodes LED (5, 6) et à activer uniquement la diode LED rayonnant dans le proche ultraviolet et/ou dans le violet (6).

2. Dispositif de rayonnement de lumière pour une résine dentaire à photopolymérisation selon la revendication 1, dans lequel il est prévu un commutateur (8) destiné à sélectionner une durée d'allumage des diodes LED de rayonnement de lumière (5, 6) à partir d'une pluralité de durées présélectionnées et/ou un commutateur de positionnement de durée pouvant positionner la durée d'allumage à une durée optionnelle.

3. Dispositif de rayonnement de lumière pour une résine dentaire à photopolymérisation selon la revendication 1 ou 2, dans lequel un filtre (10) destiné à bloquer le rayonnement ultraviolet présentant une longueur d'onde spécifique ou inférieure est agencé entre les diodes LED de rayonnement de lumière (5, 6) et un orifice de rayonnement de lumière.

4. Dispositif de rayonnement de lumière pour une résine dentaire à photopolymérisation selon l'une quelconque des revendications 1 à 3, dans lequel un rapport de puissance de sortie entre la diode rayonnant dans le bleu (5) et la diode rayonnant dans le proche ultraviolet et/ou dans le violet (6) est compris entre 9:1 et 5: 5.

## Patentansprüche

1. Lichtbestrahlungsvorrichtung, welche hauptsächlich ein Dentalphotopolymerisationskompositharz unter Verwendung einer Kombination von einem Campherchinon und einem tertiären Amin oder einem Acylphosphinoxid als Photopolymerisationskatalysator polymerisiert und härtet, **dadurch gekennzeichnet, daß** sie:
eine Blaustrahlungs-LED 5 mit einem Peak einer Lichtwellenlänge bei einer Wellenlänge von 450 nm oder mehr, aber nicht mehr als 490 nm,
eine Nahultraviolettstrahlungs- und/oder Violettstrahlungs-LED 6 mit einem Peak einer Lichtwellenlänge bei einer Wellenlänge von 370 nm oder mehr, aber weniger als 410 nm, und
einen Schaltungsänderungsschalter 7 zum gleichzeitigen Einschalten dieser zwei Arten von LEDs 5, 6 und zum Einschalten nur der Nahultraviolettstrahlungs- und/oder Violettstrahlungs-LED 6 umfaßt.

2. Lichtbestrahlungsvorrichtung für ein Dentalphotopolymerisationsharz, wie in Anspruch 1 beansprucht, wobei ein Schalter 8 zum Auswählen einer Belichtungszeit der Lichtstrahlungs-LEDs 5, 6 von einer Vielzahl von voreingestellten Zeiten und/oder ein Zeiteinstellschalter, der zum Einstellen der Belichtungszeit auf eine optionale Zeit befähigt ist, bereitgestellt ist/sind.

3. Lichtbestrahlungsvorrichtung für ein Dentalphotopolymerisationsharz, wie in Anspruch 1 oder 2 beansprucht, wobei ein Filter 10 zum Abstellen bzw. Abhalten der Ultraviolettstrahlung mit einer spezifischen Wellenlänge oder weniger zwischen den Lichtstrahlungs-LEDs 5, 6 und einem Lichtstrahlungseinlaß angeordnet ist.

4. Lichtbestrahlungsvorrichtung für ein Dentalphotopolymerisationsharz, wie in einem der Ansprüche 1 bis 3 beansprucht, wobei das Verhältnis im Output zwischen der Blaustrahlungs-LED 5 und der Nahultraviolettstrahlungs- und/oder Violettstrahlungs-LED 6 9:1 bis 5:5 beträgt.
